# EUROPEAN PATENT APPLICATION

(11) **EP 3 434 712 A1**
(43) Date of publication of application: **30.01.2019**
(21) Application number: 17182789.2
(22) Date of filing: 24.07.2017
(51) Int. Cl.: C08G 65/00, C08G 81/02, C08G 65/325, C08G 65/331, C08G 65/332, C08G 65/334, C08G 65/34

(54) **DIBLOCK COPOLYMER, A MANUFACTURING METHOD AND SUITED APPLICATIONS**

(71) Applicant: Freie Universität Berlin, 14195 Berlin (DE); President and Fellows of Harvard College, Cambridge, MA 02138 (US)
(72) Inventor: The designation of the inventor has not yet been filed
(74) Representative: Maikowski & Ninnemann Patentanwälte Partnerschaft mbB

(57) **Abstract**

The invention relates in an aspect to a diblock copolymer consisting of a first block, a second block, and a linker, wherein the second block is covalently bound to the first block by the linker. Thereby, the first block is a glycerol block comprising 1 to 10 glycerol subunits that are optionally substituted, and the second block is a superhydrophobic block comprising at least 20carbon atoms, wherein at least 40 % of all possible substitution sites of the carbon atoms are substituted by fluorine. A manufacturing method for this diblock copolymer and appropriate applications thereof are also disclosed.

## Description

### FIELD OF INVENTION

The present invention relates to a diblock copolymer according to the preamble of claim 1, to a method for manufacturing such a diblock copolymer according to the preamble of claim 9, to a droplet made of such a diblock copolymer according to the preamble of claim 12 as well as to uses of such a droplet according to the preambles of claims 14 and 15.

### BACKGROUND

US 9,012,390 B2 and US 9,498,761 B2 describe surfactants, in particular fluorosurfactants, for stabilizing aqueous or hydrocarbon droplets in a fluorophilic continuous phase. These surfactants comprise a block copolymer including a perfluorinated polyether (PFPE) block coupled to a polyethylene glycol (PEG) block via an amide bond.

From the limited library of fluorinated surfactants the perfluoropolyether-polyethylene glycol-perfluoropolyether (PFPE-PEG-PFPE) triblock copolymer is the benchmark for the formulation and stability of water-in-oil (W/O) and water-in-oil-in-water (W/O/W) emulsions, particularly for biological applications due to its biocompatibility. However, it has several limitations, for example, droplet coalescence at higher temperature, cross-contamination of microreactors (droplets), instability of droplets after a certain threshold of volume fraction, false optical read out in intensity derived fluorescence assay as droplets shrink over a short amount of time, etc. Most importantly, the molar mass dependency of both PEG and PFPE blocks for surfactant synthesis frequently leads to undesirable batch to batch variation.

To overcome some limitations of the PEG-based block copolymers, a triblock copolymer having a central linear polyglycerol block and two flanking PFPE blocks has been developed and described by Wagner et al. (Wagner, O., Thiele, J., Weinhart, M., Mazutis, L., Weitz, D. A., Huck, W. T., & Haag, R. (2016). Biocompatible fluorinated polyglycerols for droplet microfluidics as an alternative to PEG-based copolymer surfactants. Lab on a Chip, 16(1), 65-69).

Further examinations revealed that these triblock copolymers have an insufficient thermal stability.

### SUMMARY

It is an object of the present invention to provide novel compounds that can be used for forming droplets in water-in-oil (W/O) and water-in-oil-in-water (W/O/W) emulsions, wherein these droplets have at least a high thermal stability.

This object is achieved by a diblock copolymer having the features of claim 1. Such a diblock copolymer consists of a first block, a second block, and a linker. Thereby, the second block is covalently bound to the first block by the linker. The first block is a glycerol block. It comprises 1 to 10 glycerol subunits that are optionally substituted. If the number of glycerol subunits increases beyond the before-mentioned upper limit, the properties of the diblock copolymer changes so that droplets formed by such diblock copolymers do not have stability of the droplets formed by diblock copolymers complying with the definitions of claim 1.

The second block is a superhydrophobic block comprising at least 20 carbon atoms. Thereby, at least 40 % of all possible substitution sites (e.g., a methylene bridge has two substitution sites and a methyl end group of a hydrocarbon chain has three substitution sites) are substituted by fluorine. Due to such substitution by fluorine, the hydrocarbon chain of the superhydrophobic block does not only exhibit hydrophobic properties, but rather superhydrophobic properties.

The specific combination of a glycerol block having only a small number of glycerol units and superhydrophobic hydrocarbon block with a minimum of 20 carbon atoms results in a diblock copolymer having superior properties as compared to compounds known from prior art. Specifically, droplets made of these diblock copolymers show a high thermal stability so that droplets made of these diblock copolymers can be used as mini reactors for high-temperature applications, such as PCR. Likewise, the droplets can well be used for screening of small molecules, such as drugs.

The novel diblock copolymers can also be denoted as glycerol-based fluorosurfactants. They can be applied to stabilize W/O and W/O/W emulsions. Non-limiting examples of applications of these emulsions include single cell DNA analysis by polymerase chain reaction (PCR), evolution of enzyme function, cell cytotoxicity test in microdroplets, and high throughput screening of low molecular weight molecules. Surprisingly, droplets formed by the claimed diblock copolymer showed almost no cross-contamination by reagents contained within the droplets. Expressed in other words, the droplets did on the one hand not release (to a significant extent) reagents contained in their interior and did on the other hand not take up (to a significant extent) reagents from their surroundings. This extremely low cross-contamination capability makes droplets formed by the claimed diblock copolymer particularly appropriate for encapsulating small molecules used in the water phase.

Essentially, the fluorophilic compounds for the tail of this surfactant (building the superhydrophobic block) can be any available perfluoropolyether that contains a carboxylic group at its terminus. Non-limiting examples are fluorinated alkyl chains of any size and/or length with linear, hyperbranched, branched, saturated, unsaturated, dendritic, cyclic, homobifunctional, or heterobifuctional architectures. Appropriate PFPE polymers for building the superhydrophobic block are manufactured by DuPont under the trade names Krytox® 157 FSH, Krytox® 157 FSM, and Krytox® 157 FSL.

On the other hand, the head group (building the glycerol block) in the novel amphiphilic surfactants is an oligo-glycerol derivative, wherein the number of glycerol units varies from 1 to 10. The head group of the surfactant make the surfactant water soluble, hygroscopic and biocompatible. Essentially, the hydrophilic oligo-glycerol head group can be either one of its enantiomers (R,R and S,S), a mixture of enantiomers, meso-form or a mixture of individual stereoisomers. The hydroxyl groups in the head groups of the surfactant can be pre-modified and/or post-modified to render it soluble in organic solvent. Any anionic, cationic, non-ionic, and zwitterionic compound can be coupled to the head group during pre-/post- modification.

The head group (glycerol block) is coupled to the fluorophilic tail (superhydrophobic block) *via* a linker forming any covalent bond. More specifically, the coupling chemistry can include any kind of click chemistry, amide bond formation, esterfication, Friedel-Crafts alkylation, but is not limited to these synthesis reactions.

As mentioned, the molar mass dependence of the benchmark surfactant renders the formulation uncontrolled from batch to batch. If no stoichiometric reaction is achieved, a mixture of di-block and tri-block co-polymers, as well as homo-polymers of both hydrophilic and fluorophilic groups results. On the contrary, even though there is a distribution of molar mass of commercially available PFPE, the flexibility on stoichiometric reaction conditions makes the synthesis of diblock copolymers as described herein not only more controlled but also superior over the synthesis of PFPE-PEG-PFPE surfactants. Thereby, an aqueous or organic medium can be used as dispersed phase to solubilize the glycerol block. The fluorinated oils HFE 7500 (hydrofluoroether 7500, molecular weight 414 g/mol) and FC40 can be very well used to prepare the continuous phase to solubilize the superhydrophobic block of the diblock copolymer.

The glycerol subunits of the glycerol block can be substituted by any residues that are usually used for substituting polyglycerol, such as thiol, methyl, ethyl, amine, methoxy, ethoxy, methylethylether, phosphate, sulfate, phosphate choline, and choline phosphate residues. In an embodiment, however, the glycerol subunits of the glycerol block are non-substituted, i.e., they carry only hydrogen and hydroxyl residues. A diblock copolymer with such non-substituted glycerol subunits in the glycerol block exhibits higher biocompatibility as compared to a diblock copolymer with substituted glycerol subunits.

While a diblock copolymer comprising only a single glycerol subunit (monoglycerol) or two glycerol subunits (diglycerol) is principally possible, the positive properties of the diblock copolymer are particularly well achieved if an oligoglycerol comprising 3 to 10 glycerol subunits makes up the first block. Therefore, in an embodiment, the glycerol block comprises 3 to 10 optionally substituted glycerol subunits, such as 3, 4, 5, 6, 7, 8, 9 or 10 glycerol subunits. Thereby, 3 or 7 glycerol subunits are particularly appropriate to build up the glycerol block. Thus, the oligoglycerol can, in an embodiment, comprise 3 to 7 glycerol subunits.

In an embodiment, the oligoglycerol is a branched oligoglycerol. It turned out that a diblock copolymer comprising a branched oligoglycerol can particularly well stabilize droplets made of such a diblock copolymer. Thereby, the term "branched" refers to an arrangement in which the individual glycerol subunits are not linearly connected to each other. Furthermore, the superhydrophobic block is connected to the glycerol block by a carbon atom of the glycerol block that is not a terminal carbon atom of any of its glycerol subunits.

In an embodiment, the glycerol block corresponds to general formulae (I), (II), (III), (IV), (V), (VI), (VII), or (VIII):

| | |
|---|---|
| 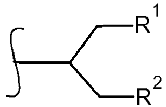 | (I) |
| 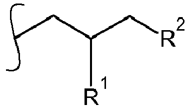 | (II) |

| | |
|---|---|
| 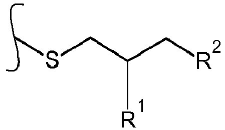 | (III) |
| 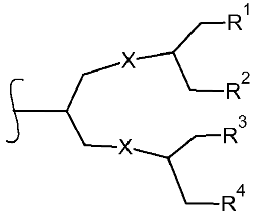 | (IV) |
| 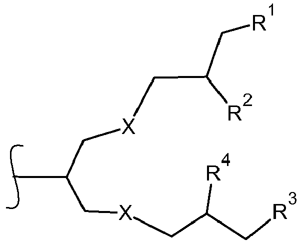 | (V) |
| 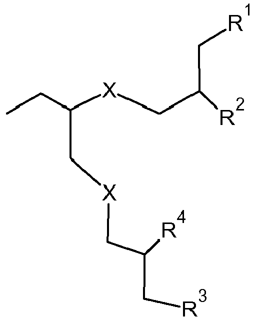 | (VI) |

| | |
|---|---|
| 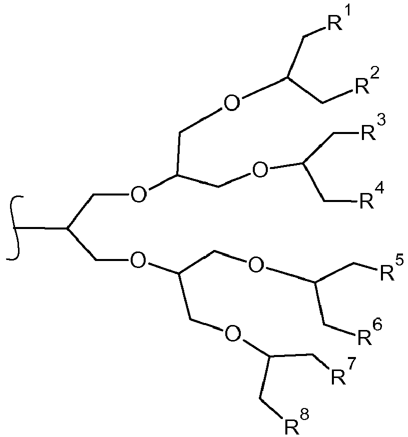 | (VII) |
| 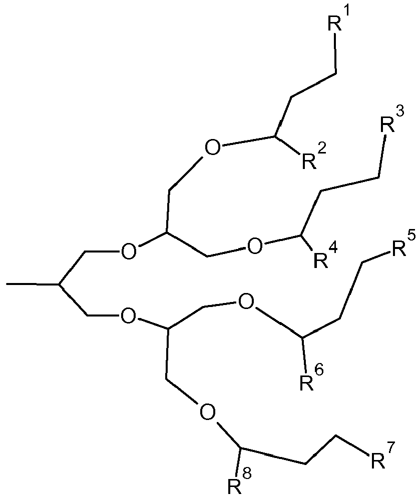 | (VIII) |

Thereby, R¹, R², R³, R⁴, R⁵, R⁶, R⁷, R⁸ denote independently from each other, -SH, -CH₃, -CH₂CH₃, -NH₂, -OH, -OCH₃, -OCH₂CH₃, -OCH₂OCH₂CH₃, -NH₃⁺, -OPO₃⁻(CH₂)₂N⁺(CH₃)₃, -(CH₃)₂N⁺(CH₂)₂OPO₂⁻(OCH₃), -(CH₃)₂N⁺(CH₂)₂OPO₂⁻(OH), or-OSO₃⁻.

Furthermore, X means O or S. O is particularly appropriate.

Formulae (I) to (III) describe a G0 glycerol (derivative). Formulae (IV) to (VI) describe a branched G1 oligoglycerol (derivative) comprising three glycerol residues. Formulae (VII) and (VIII) describe a branched G2 oligoglycerol (derivative) comprising seven glycerol residues.

In an embodiment, the superhydrophobic block comprises at least 22, 24, 26, 28, 30, 35, 40, 45, or 50 carbon atoms. In an embodiment, it comprises 20 to 50 carbon atoms, in particular 22 to 45, in particular 24 to 40, in particular 26 to 35, in particular 28 to 30 carbon atoms. These carbon atoms can be arranged in a single hydrocarbon chain. However, such an arrangement is not mandatory. Rather, it is possible that the carbon atoms of the superhydrophobic block are arranged in more than one hydrocarbon chain, such as two or three hydrocarbon chains. Thereby, the number of chains depends on the chemical nature of the linker used to couple the second block to the first block.

In an embodiment, the superhydrophobic block comprises two carbon-containing chains (such as hydrocarbon chains) that are bound to the same linker. Thereby, these carbon-containing chains are not covalently cross-linked to each other. Rather, the only covalent connection between the first carbon-containing chain and the second carbon-containing same is via the linker to which both chains are covalently bound.

In an embodiment, at least 45%, in particular at least 50%, in particular at least 55%, in particular at least 60%, in particular at least 65%, in particular at least 70%, in particular at least 75%, in particular at least 80%, in particular at least 85%, in particular at least 90%, in particular at least 95% and very particular all substitution sites of the carbon atoms of the carbon-containing chain of the superhydrophobic block are substituted by fluorine. In an embodiment, 40 % to 100 %, in particular 45 % to 95 %, in particular 50 % to 90 %, in particular 55 % to 85 %, in particular 60 % to 80 %, in particular 65 % to 75 % of all substitution sites of the carbon atoms of the carbon-containing chain of the superhydrophobic block are substituted by fluorine.

If the superhydrophobic block comprises more than one carbon-containing chain, all carbon-containing chains have, in an embodiment, the same number of carbon atoms and, in another embodiment, the same overall chemical structure.

In an embodiment, the superhydrophobic block comprises a perfluoroether (PFPE) residue, in particular a PFPE residue as described in US 9,012,390 B2, as long as it complies with constraints of a minimum number of carbon atoms as defined above. For this purpose, US 9,012,390 B2 is incorporated herein by reference.

In an embodiment, the superhydrophobic block comprises a residue according to general formulae (IX) or (X):

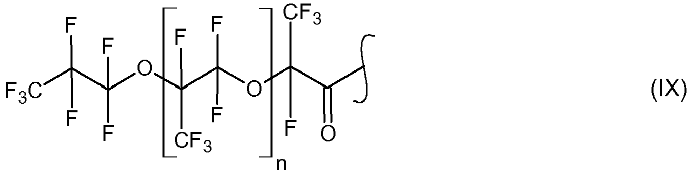

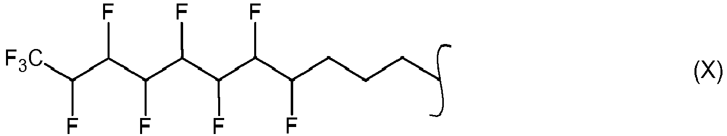

Thereby, n is an integer between 10 and 50.

In an embodiment, n is 10 to 20, in particular 10 to 18, in particular 10 to 16, in particular 10 to 14. In another embodiment, n is 20 to 30, in particular 22 to 28, in particular 24 to 26. In another embodiment, n is 30 to 50, in particular 32 to 48, in particular 34 to 46, in particular 36 to 44, in particular 38 to 42.

In an embodiment, the linker is at least one chosen from the group consisting of amines, amides, oxygen (i.e. an ether bridge), and an alkyl residue having 1 to 20 carbon atoms, wherein a hydrocarbon chain of the alkyl residue can be interrupted by one or more oxygen, nitrogen and/or sulfur atoms and/or can be substituted by a group comprising one or more oxygen, nitrogen and/or sulfur atoms.

In an aspect, the instant invention also relates to a method for manufacturing a diblock copolymer as defined above. This manufacturing method comprises a step of reacting an oligoglycerol amine comprising protected hydroxyl groups with a perfluorinated polyether acid chloride in an organic solvent, and deprotecting the hydroxyl groups of the obtained diblock copolymer. The organic solvent comprises, in an embodiment, a hydrofluoroether such as HFE-7100 or HFE-7500. The organic solvent comprises, in an embodiment, dichloromethane. A mixture of a hydrofluoroether and dichloromethane is particularly appropriate as organic solvent. The deprotection can be carried out, in an embodiment, under acidic conditions. An acetal protecting group is an appropriate protecting group. Appropriate reaction temperatures lie in a range between 40 and 55 °C, in particular between 45 and 50 °C.

In an embodiment, the oligoglycerol amine comprising protected hydroxyl groups is manufactured by mesylating an oligoglycerol comprising protected hydroxyl groups and at least one unprotected hydroxyl group to introduce a mesyl group into the oligoglycerol. An appropriate mesylation compound is methane sulfonyl chloride. Appropriate reaction temperatures lie in a range between 0 °C and 30 °C, in particular between 10 and 25 °C. The mesyl group is then replaced by an azide group. This is done, in an embodiment, by reacting the mesylated oligoglycerol with an azide compound such as sodium azide. Dichloromethane is an appropriate solvent for such an azidation. Appropriate reaction temperatures lie in a range between 50 and 70 °C, in particular between 55 and 65 °C. Finally, the azide group is hydrogenated to obtain the oligoglycerol amine comprising protected hydroxyl groups. Hydrogenation is carried out, in an embodiment, with the help of a catalyst such as a palladium catalyst or a palladium/carbon catalyst. Methanol is an appropriate solvent for such a hydrogenation. Appropriate reaction temperatures lie in a range between 0 °C and 30 °C, in particular between 10 and 25 °C.

In an embodiment, the perfluorinated polyether acid chloride is manufactured by reacting a perfluorinated polyether with oxalyl chloride in an organic solvent. The organic solvent comprises, in an embodiment, a hydrofluoroether such as HFE-7100 or HFE-7500. The organic solvent comprises, in an embodiment, dichloromethane. A mixture of a hydrofluoroether and dichloromethane is particularly appropriate as organic solvent. This organic solvent can be identical or different to the organic solvent used for reacting the oligoglycerol amine comprising protected hydroxyl groups with the perfluorinated polyether acid chloride. Appropriate reaction temperatures lie in a range between 0 °C and 30 °C, in particular between 10 and 25 °C.

In another aspect, the present invention relates to a droplet made of a plurality of diblock copolymers according to the preceding explanations. Such a droplet typically comprises a single layer of diblock copolymers. If this droplet is formed in an oily medium, such as a perfluorinated oil, e.g., a short-chained perfluorinated oil, the second block (superhydrophobic block) is arranged to an outside of the droplet, wherein the first block (glycerol block) is arranged to an inside of the droplet. Then, a hydrophilic phase, such as an aqueous phase, can be incorporated within the interior of the droplet. To give an example, a buffer system in an aqueous phase such as water can be present in the inside of a corresponding droplet. A culture medium is also a suited aqueous phase to be incorporated into a corresponding droplet.

In an embodiment, the droplet does not comprise only diblock copolymers of the same structure, but structurally different diblock copolymers. To give an example, the droplets may comprise a first diblock copolymer comprising a low molecular weight PFPE and a second diblock copolymer comprising a medium molecular weight PFPE. Similarly, the droplet might comprise a second diblock copolymer comprising a medium molecular weight PFPE and a third diblock copolymer comprising a high molecular weight PFPE. Likewise, if a mixture of a first diblock copolymer comprising a low molecular weight PFPE and a third diblock copolymer comprising a high molecular weight PFPE is also possible. Furthermore, a droplet may be composed of more than two structurally different diblock copolymers. To give an example, such droplets might comprise a first diblock copolymer, a second diblock copolymer and a third diblock copolymer according to the preceding definitions.

The term "low molecular weight" with respect to the PFPE residue of the diblock copolymer relates, in an embodiment, to a PFPE residue (or superhydrophobic block) having a molecular weight of 1500 to 3000 g/mol. If the superhydrophobic block corresponds to general formula (IV), such molecular weight is achieved if n is 10 to 16 (or an integer between these thresholds).

The term "medium molecular weight" with respect to the PFPE residue of the diblock copolymer relates, in an embodiment, to a PFPE residue (or superhydrophobic block) having a molecular weight of 3500 to 5000 g/mol. If the superhydrophobic block corresponds to general formula (IV), such molecular weight is achieved if n is 22 to 28 (or an integer between these thresholds).

The term "high molecular weight" with respect to the PFPE residue of the diblock copolymer relates, in an embodiment, to a PFPE residue (or superhydrophobic block) having a molecular weight of 5500 to 8000 g/mol. If the superhydrophobic block corresponds to general formula (IV), such molecular weight is achieved if n is 34 to 46 (or an integer between these thresholds).

In an aspect, the invention relates to an emulsion of an aqueous dispersed phase, a continuous phase comprising a fluorinated oil, and a surfactant comprising a diblock copolymer according to the preceding explanations. Typically, the surfactant serves for droplet formation and/or stabilization of the dispersed phase in the continuous phase.

In an embodiment, the dispersed phase has an average diameter bigger than or equal to about 20 nm and less than or equal to about 200 microns.

In an embodiment, the dispersed phase has an average diameter of no more than about 200 microns.

In an embodiment, the dispersed phase has an average diameter bigger than or equal to about 20 nm.

In an embodiment, the dispersed phase comprises an aqueous solution comprising a biological molecule. In an embodiment, the biological molecule comprises a nucleic acid. In an embodiment, the biological molecule comprises an oligonucleotide.

In an embodiment, the dispersed phase comprises at least one of buffers, salts, nutrients, therapeutic agents, drugs, hormones, antibodies, analgesics, anticoagulants, antiinflammatory compounds, antimicrobial compositions, cytokines, growth factors, interferons, lipids, oligonucleotides polymers, polysaccharides, polypeptides, protease inhibitors, cells, nucleic acids, RNA, DNA, vasoconstrictors or vasodilators, vitamins, minerals, and stabilizers.

In an aspect, the present invention also relates to a method for performing a chemical and/or biological reaction in the dispersed phase according to the preceding explanations.

In an aspect, the present invention also relates to the use of the droplets according to the preceding explanations for performing a PCR in an interior of the droplets. As outlined above, the droplets formed by the instantly claimed diblock copolymers have a very high thermal stability. Therefore, they can well be used as mini reactors for performing a PCR in their interior. When heating up an emulsion of corresponding droplet to temperatures being typical for a PCR (up to ca. 98 °C), the droplets remain stable and do not disintegrate. Thus, all components being composed within the interior of the droplets remain in the interior and can be used for reactions such like the reactions to be typically performed during a PCR (i.e., denaturation, annealing and extension).

In an aspect, the instant invention also relates to a method for performing a PCR in an interior of a droplet according to the preceding explanations.

In an aspect, the instant invention also relates to the use of a droplet according to the preceding explanations for testing in vitro an effect of a substance on a cell incorporated in the droplet. Thereby, the substance can be, in an embodiment, a pharmaceutically active substance, such as a pharmaceutically active small molecule. It is also possible to incorporate more than one substance into the droplet. Thus, a combination of pharmaceutically active substances can be incorporated. For such a test of one or more substances in a single droplet, a cell like a tumor cell can be incorporated together with culture medium and the pharmaceutically active substance within an interior of the droplet formed by at least one type of diblock copolymer according to the preceding explanations. Afterwards, the cell viability within the droplet can be determined and compared with the cell viability of a cell incorporated in a droplet containing the same culture medium in its interior, but no pharmaceutically active substance. By such an experimental setup, which can be realized by a plurality of microfluidic systems arranged in parallel for producing droplets with incorporated cells and optionally a substance to be tested, a high number of pharmaceutically active substances can be tested with respect to their effect on the viability of cells, regulation of gene expression, alteration of genotype and phenotype of cells (or other properties).

In an aspect, the instant invention relates to a method for testing an effect of the substance on a cell incorporated in a droplet comprising a plurality of diblock copolymers according to the preceding explanations.

All embodiments described herein can be combined in any desired way. Furthermore, embodiments described with respect to the diblock copolymer can be transferred to any of its manufacturing method, the droplet made of a plurality of diblock copolymers and the uses of the droplet, and vice versa. Likewise, embodiments of any described subject matter can be transferred to any other subject matter described herein.

### BRIEF DESCRIPTION OF THE DRAWINGS

Further details of aspects of the present invention will be explained with respect to exemplary embodiments and accompanying Figures. In the Figures:
- Figure 1A: shows droplets made from a PFPE-PEG-PFPE triblock copolymer according to US 9,012,390 B2 after performing a PCR at 95 °C;
- Figure 1B: shows droplets made from a PFPE-LPG(OMe)-PFPE triblock copolymer according to Wagner et al. after performing a PCR at 95 °C;
- Figure 1C: shows droplets made from an embodiment of a diblock copolymer as described herein after performing a PCR at 95 °C;
- Figure 2A: shows a size distribution of the droplets depicted in Figure 1A;
- Figure 2B: shows a size distribution of the droplets depicted in Figure 1B;
- Figure 2C: shows a size distribution of the droplets depicted in Figure 1C;
- Figure 3: shows different panels depicting differently composed droplets with and without fluorescence dye directly after generating the droplets (0 hours) and after an incubation period of 72 hours;
- Figure 4: shows a plot on the cell viability of a leukemia cell line incorporated within droplets of different composition;
- Figure 5: shows a reaction scheme of an embodiment of a method for synthesizing a protected oligoglycerol amine;
- Figure 6: shows a reaction scheme of an embodiment of a method for chlorinating a perfluorinated polyether; and
- Figure 7: shows a reaction scheme of an embodiment of a method for manufacturing a diblock copolymer starting from a protected oligoglycerol amine and a perfluorinated polyether chlorate.

### DETAILED DESCRIPTION

The Figures will now be explained with respect to exemplary embodiments.

### Example 1: Temperature stability

The diblock copolymers described herein have the capability to stabilize emulsions even at high temperatures which can be essential for performing assays in droplets. For example, polymerase chain reaction (PCR) is the most widely used method for DNA amplification which is an indispensable constitute for drop-based biological assays. However, PCR requires as high as 98 °C incubation temperature in amplification cycles. At this high temperature, emulsions with PCR reagents stabilized by PFPE-PEG-PFPE triblock copolymers known from prior art fail to remain stable and coalesce.

Monodisperse PCR reagent droplets having a diameter of about 75 µm stabilized by 2% (w/w) surfactant in HFE 7500 solution were tested. The following surfactants were used for building up the droplets:
a) PFPE-PEG-PFPE triblock copolymer as described in US 9,012,390 B2 (Krytox-PEG-Krytox);
b) PFPE-LPG(OMe)-PFPE triblock copolymer as described in Wagner et al. (Krytox-LPG(OMe)-Krytox);
c) PFPE-PG diblock copolymer according to the following formula (XI) (G1-Krytox; LG1): 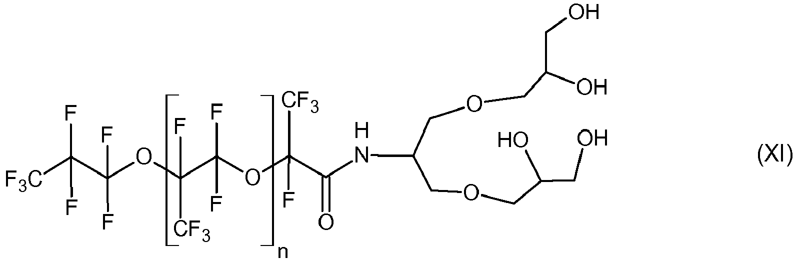 with n = 10 to 16

The used PCR kit was the Phusion kit from Thermo Fisher with detergent-free buffer. Lambda Phage genome fragmented and tagged by Nextera Kit was used as template.

After performing 35 PCR cycles with 98 °C denaturation temperature (whole cycling conditions: 98 °C for 30 s, then 35 cycles of: 98 °C for 7 s, 60 °C for 30 s, 72 °C for 20 s. After cycling, 72 °C for 10 min), about 25% of emulsions stabilized by PFPE-PEG-PFPE merged into a water layer while only 10% of emulsion stabilized by LG1 merged into water layer.

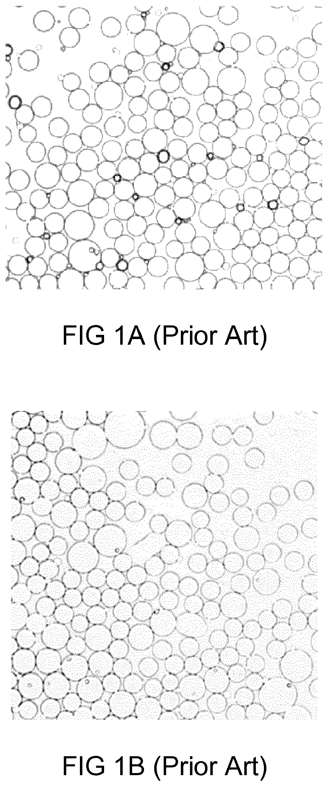

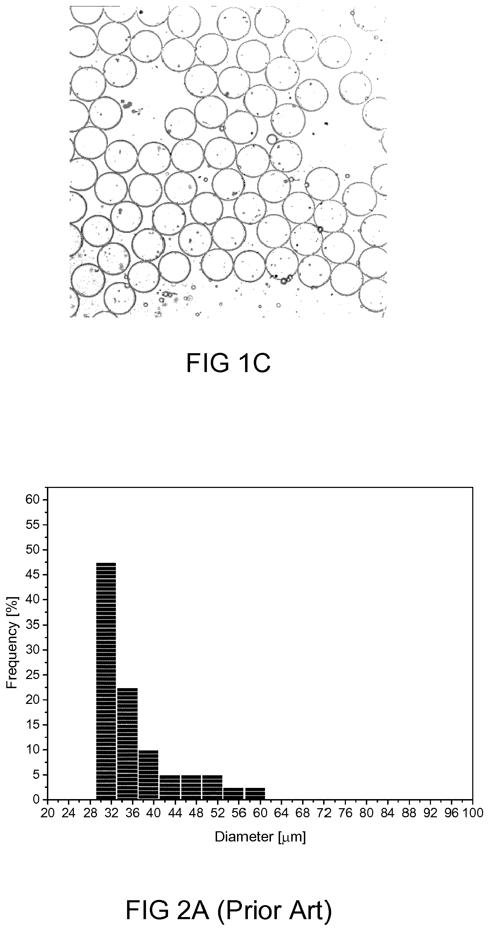

The droplets that survived the PCR cycles were examined under microscope. Droplets stabilized by PFPE-PEG-PFPE (Figure 1A) and PFPE-LPG(OMe)-PFPE (Figure 1 B) were no longer monodisperse. This clearly indicates that they were destabilized due to the temperature treatment. Droplets stabilized by LG1 remained, however, monodisperse (Figure 1C).

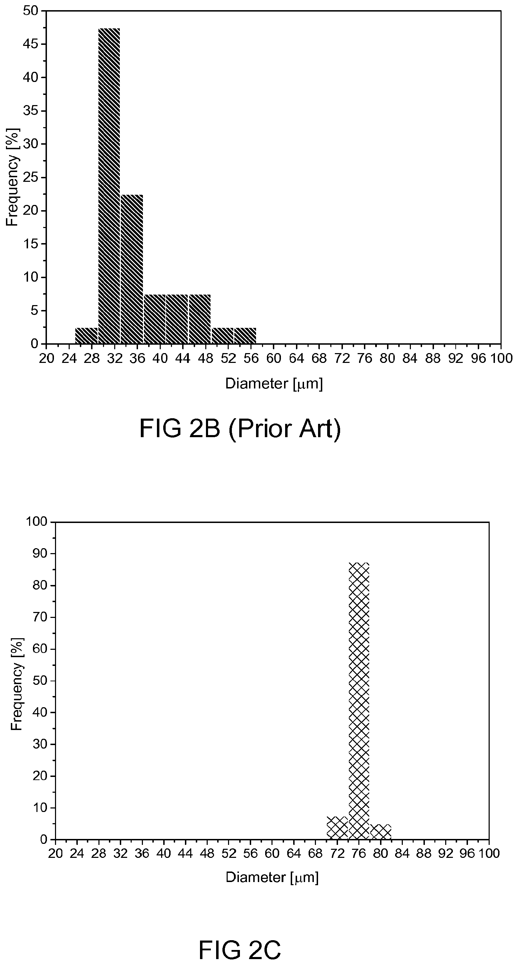

The size of the droplets stabilized by different surfactants was examined in more detail by measuring the sizes of the droplets depicted in Figures 1A to 1C and assigning a size distribution to the corresponding droplets. The results are shown in Figures 2A to 2C. PFPE-PEG-PFPE (Figure 2A) and PFPE-LPG(OMe)-PFPE (Figure 2B) show a broad size distribution in a diameter range of approximately 25 to 60 µm with a highest incidence in both cases at 29 to 33 µm. In contrast, the droplets stabilised by LG1 only show a very narrow size distribution in a diameter range of 70 to 82 µm with a highest incidence at 74 to 78 µm (Figure 2C).

### Example 2: Cross-talking among droplets

As a superb platform for precisely miniaturizing and compartmentalizing chemical assays, droplet-based microfluidics is of particular interest in a number of biotechnological applications. The technology essentially utilizes water-in-oil emulsions as droplet microreactors, typically at nanoliter or picoliter scale. The extent of application is, however, limited by the so-called cross-talking between droplets. Cross-talking denotes molecular transport between droplets, leading to an inaccuracy of the droplet-based assays. As driven by the thermodynamic disequilibrium of aqueous/oil interfaces, the cross-talking phenomenon is also affected by the type of surfactant used to stabilize the emulsion.

To test the cross-talking phenomenon, two kinds of aqueous droplets were made and incubated for 72 hours: smaller droplets incorporating fluorescent dye dissolved in phosphate-buffered saline (PBS) (positive droplets) and larger droplets incorporating only PBS without dye (negative droplets). These two types of droplets were prepared with different droplet-stabilizing surfactants using a surfactant concentration of 2% (w/w) in a HFE 7500 solution.

Images of the droplets were taken immediately after generating the droplets (0 hours) and after an incubation period of 72 hours using a confocal microscope.

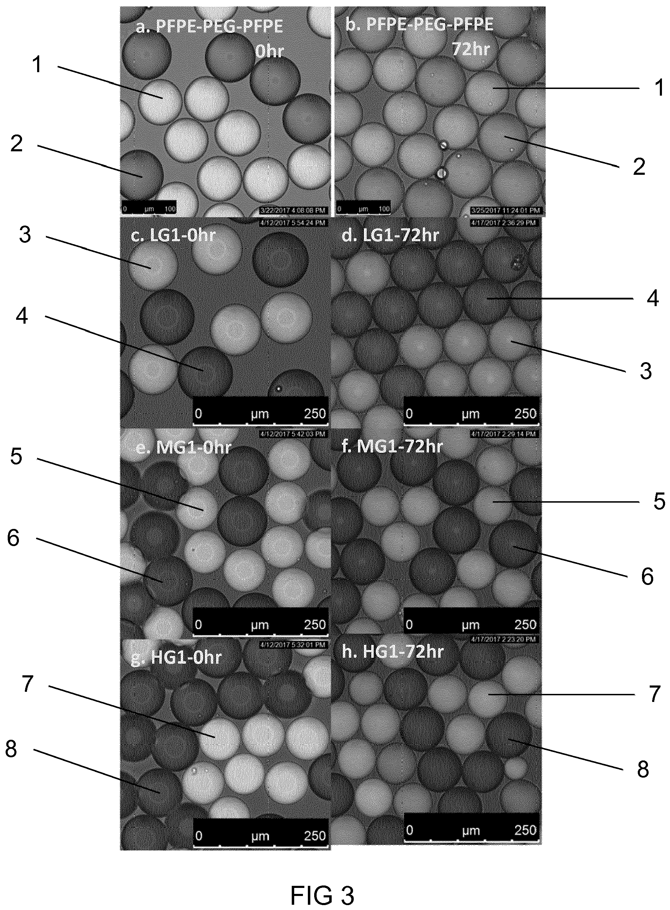

Panels a and b of Figure 3 show droplets stabilized by PFPE-PEG-PFPE. At 0 hours, the positive droplets 1 show a strong green fluorescence (light grey in Figure 3), whereas the negative droplets 2 do not show any green fluorescence and all (dark grey in Figure 3). After an incubation period of 72 hours, the fluorescence of the previously positive droplets 1 diminished, whereas the fluorescence of the previously negative droplets 2 increased. Rather, an almost equal distribution of the fluorescence between previously positive droplets 1 and previously negative droplets 2 could be observed.

Panels c and d of Figure 3 show droplets stabilized by LG1. At 0 hours, the positive droplets 3 show a strong green fluorescence (light grey in Figure 3), whereas the negative droplets 4 do not show any green fluorescence and all (dark grey in Figure 3). After an incubation period of 72 hours, the fluorescence of the positive droplets 3 almost remained unchanged, whereas the negative droplets 4 did still not show a significant fluorescence. This clearly indicates that LG1-stabilized droplets decrease the extent of cross-talking between individual droplets. Thus, if a substance is incorporated into an LG1-stabilized droplet, it remains within this droplet and is not transported to another droplet to a significant extent.

Panels e and f of Figure 3 show droplets stabilized by MG1. MG1 has a similar structure like LG1 but a higher molecular weight than LG1. It corresponds to formula (XII): 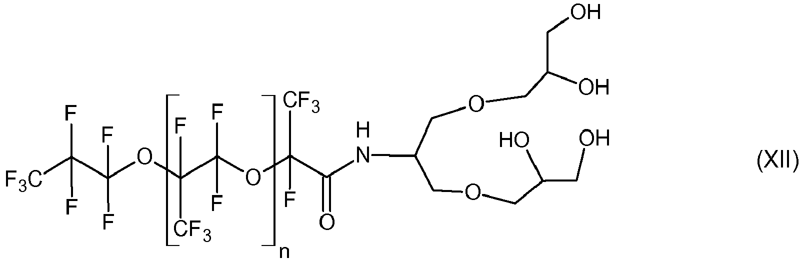 with n = 22 to 28

At 0 hours, the positive droplets 5 show a strong green fluorescence (light grey in Figure 3), whereas the negative droplets 6 do not show any green fluorescence and all (dark grey in Figure 3). After an incubation period of 72 hours, the fluorescence of the positive droplets 5 almost remained as strong as 0 hr, whereas the negative droplets 6 did still not show any fluorescence. This clearly indicates that also MG1-stabilized droplets do not show cross-talking between individual droplets. Thus, if a substance is incorporated into an MG1-stabilized droplet, it remains within this droplet and is not transported to another droplet.

Panels g and h of Figure 3 show droplets stabilized by HG1. HG1 has a similar structure like LG1 and MG1 but an even higher molecular weight than MG1. It corresponds to formula (XIII): 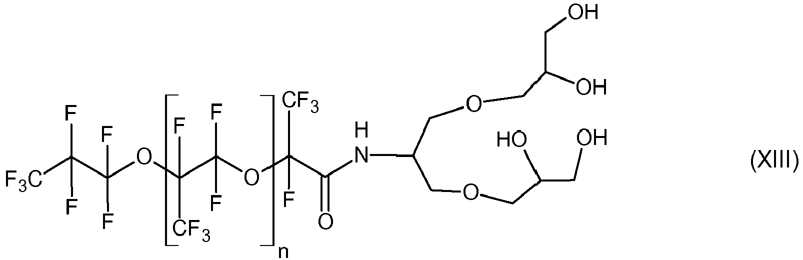 with n = 34 to 46

At 0 hours, the positive droplets 7 show a strong green fluorescence (light grey in Figure 3), whereas the negative droplets 8 do not show any green fluorescence and all (dark grey in Figure 3). After an incubation period of 72 hours, the fluorescence of the positive droplets 7 almost remained as strong as 0 hr, whereas the negative droplets 8 did still not show any fluorescence. This clearly indicates that also HG1-stabilized droplets do not show cross-talking between individual droplets. Thus, if a substance is incorporated into an HG1-stabilized droplet, it remains within this droplet and is not transported to another droplet.

### Example 3: Superior biocompatibility

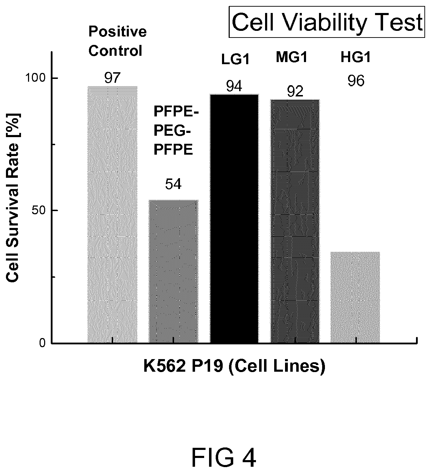

The biocompatibility of LG1, MG1, and HG1 was assessed by means of a cell viability test. In the test, individual cells of the leukemia cell line K562 were encapsulated in the droplets (100µm). Fluorinated oil was used as the continuous phase and cell culture medium (DMEM) supplemented with 10% FBS was used as the aqueous phase. After 3 days of incubation, the droplets were destabilized by perfluorooctanol (PFO) and the cell viability was evaluated using a "live/dead staining kit". As shown in Figure 4, the cell viability in droplets stabilized by PFPE-PEG-PFPE (comparative example; negative control) was found to be 54 %, whereas the cell viability in LG1-, MG1-, and HG1-stabilized droplets was above 90 % for all three types of droplets stabilized by a diblock copolymer as described herein. The cell viability in LG1-, MG1-, and HG1-stabilized droplets almost achieved a viability rate of 97 % observed for cells incubated for 3 days in cell culture medium (DMEM) supplemented with 10% FBS (comparative example; positive control). No droplets were formed in this positive control.

### Example 4: Synthesis of an embodiment of a diblock copolymer

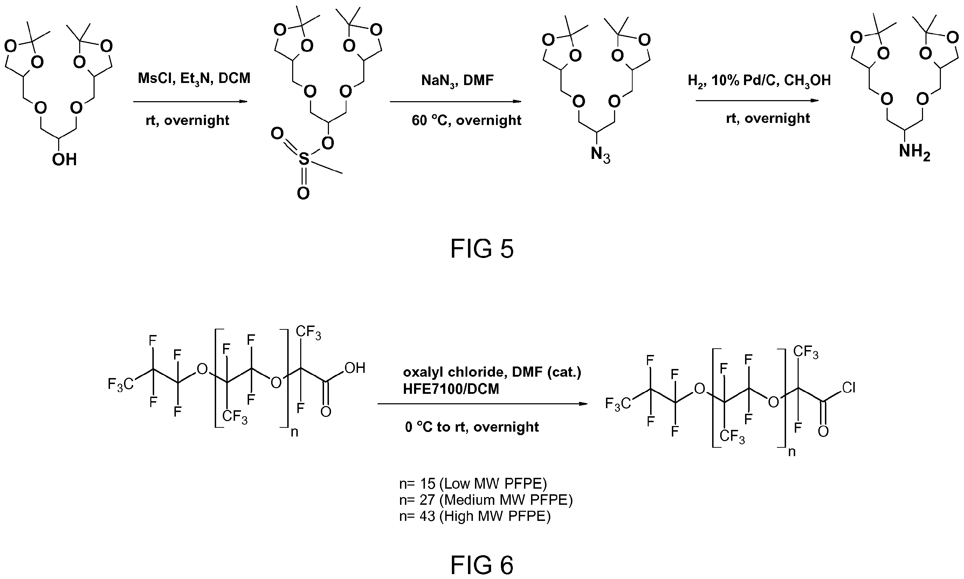

First, a protected oligoglycerol amine was manufactured according to the reaction scheme depicted in Figure 5. The starting material was an acetal protected [G1] polyglycerol dendron hydroxy that was dried in high vacuum at 60 °C overnight. Thereafter, G1 dendron-N₃ was prepared in a two-step process. First, the mesylation of the hydroxyl groups was carried out using 1.5 equivalent of methane sulfonyl chloride (MsCl) and two equivalents of triethyl amine in dichloromethane (DCM) at 0 °C to room temperature (RT). The mesylated compound was extracted in DCM and then dried under high vacuum (HV). Afterwards, the dried compound was dissolved in dimethylformamide (DMF) under argon, and NaN₃ (3 eq.) was added to the above reaction mixture. Substitution of the mesyl group with azide was carried out overnight at 70 °C. The final compound was obtained in 95% yield after extraction in DCM followed by drying in HV.

Finally, G1 dendron-N₃ was dissolved in methanol and hydrogenation of azide (-N₃) functional groups was conducted in presence of palladium catalyst (10% Pd/Charcoal) in a reactor with vigorous stirring for two days. The reduced compound was filtered off using a bed of cellite^{®}545. Subsequently, the solvent was removed and the residue was purified by column chromatography on silica gel using a hexane:isopropanol mixture as eluent. The thus purified product was obtained with 75% yield after drying in HV.

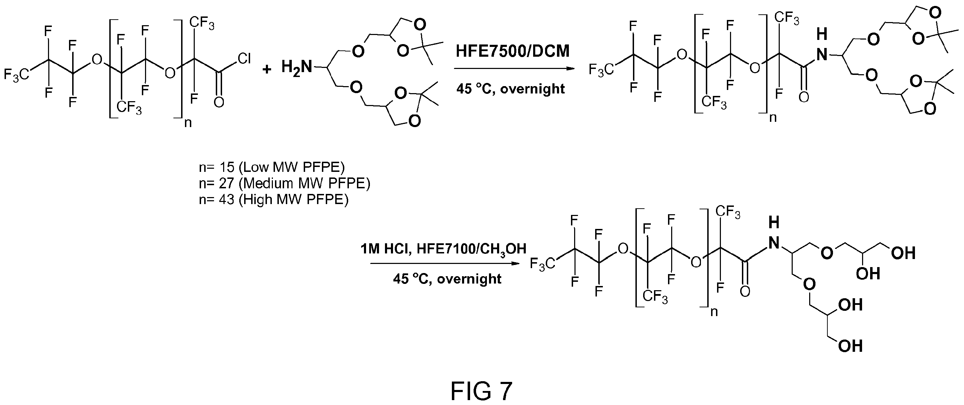

Separate from this, a perfluorinated polyether (PFPE) was activated to acid chloride overnight by reacting it with oxalyl chloride according to the reaction scheme depicted in Figure 6. The protected oligoglycerol amine synthesized according to the reaction scheme depicted in Figure 5 and the PFPE-COCI synthesized according to the reaction scheme depicted in Figure 6 were then reacted according to the reaction scheme in Figure 7 so that per-fluorinated diblock-copolymer based surfactants were synthesized in a three-step process. An amide bond is formed in the final step, wherein the amine group acts as linker between the PFPE block and the oligoglycerol block.

Firstly, a catalytic amount of DMF and oxalyl chloride (10 eq.) was added under argon atmosphere to a solution of perfluorinated polyethers (PFPE) in HFE-7100 to convert the terminal carboxylic group of PFPE to acid chloride. In the meantime, the argon filled balloon was removed and the outlet of a Schleck flask was connected to a Schleck line to let the gases escape during the reaction. After several minutes, the outlet valve was closed and an argon balloon was introduced again. The reaction mixture was stirred overnight at RT. Next, HFE-7100, DMF, and all volatile gases were removed under HV using an addition cold trap. The PFPE species Krytox 157 FSH, Krytox 157 FSM, and Krytox 157 FSL were used as high, medium, and low molecular weight block, respectively, and all of them were activated to acid chloride in a similar fashion.

Next, the viscous PFPE acid chloride was diluted adding small amount of HFE-7100 oil. Then a freshly prepared solution of G1 dendron-NH₂ (dried under HV at 50 °C) (1.07 eq. to PFPE acid chloride) in dry DCM was mixed with HFE-7100 oil in a ratio of 1:0.75. Later, dropwise addition of G1 dendron-NH₂ solution into the activated Krytox compound was performed under inert condition using a dropping funnel over a period of ∼1 h. The reaction mixture was then refluxed overnight at 45-50 °C. The crude product was transferred into a one neck round bottom flask. DCM was evaporated under reduced pressure followed by removal of HFE-7100 in HV using an additional cold trap. Lastly, the dried viscous compound was dissolved in a mixture of HFE-7100 and methanol solution and deprotection acetal groups was carried out overnight at 50 °C adding 0.5-1 mL of 1 M HCl solution to the above reaction mixture. The thus prepared high, medium, and low MW diblock-copolymer surfactants (having 15, 27 or 43 repeating units) were obtained with a yield of 75-85% after drying in HV.

## Claims

1. Diblock copolymer consisting of a first block, a second block, and a linker, wherein the second block is covalently bound to the first block by the linker, wherein
• the first block is a glycerol block comprising 1 to 10 glycerol subunits that are optionally substituted, and
• the second block is a superhydrophobic block comprising at least 20 carbon atoms, wherein at least 40 % of all possible substitution sites of the carbon atoms are substituted by fluorine.

2. Diblock copolymer according to claim 1, wherein the glycerol block comprises an optionally substituted oligoglycerol having 3 to 10 glycerol subunits.

3. Diblock copolymer according to claim 2, wherein the oligoglycerol is a branched oligoglycerol.

4. Diblock copolymer according to any of the preceding claims, wherein the glycerol block corresponds to any of general formulae (I) to (VIII) 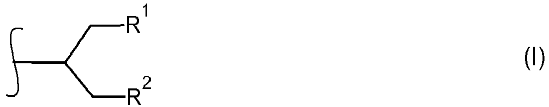 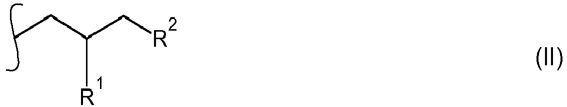 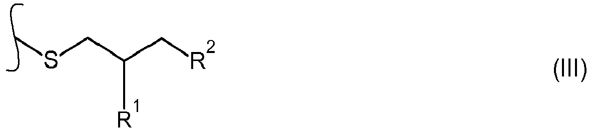 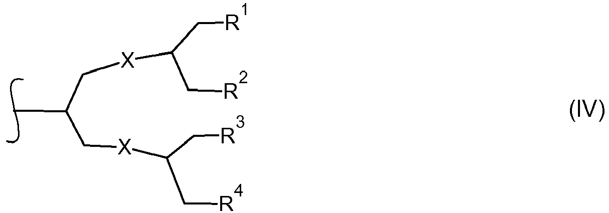 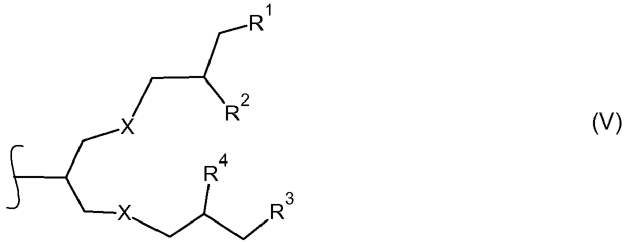 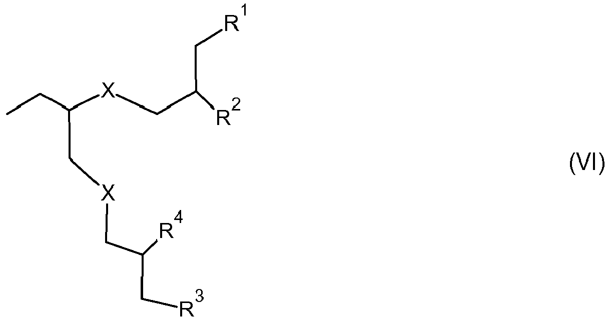 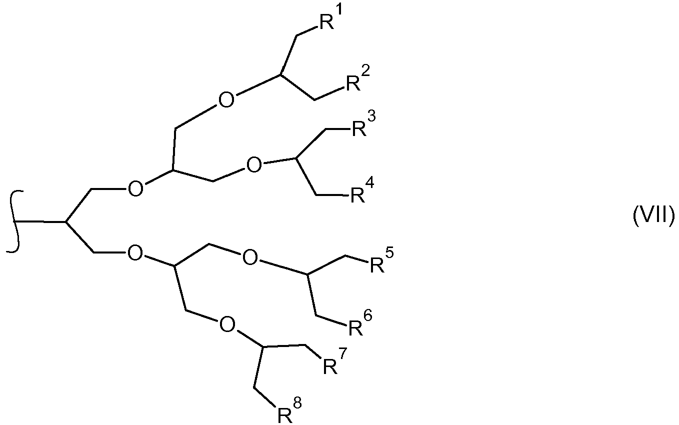 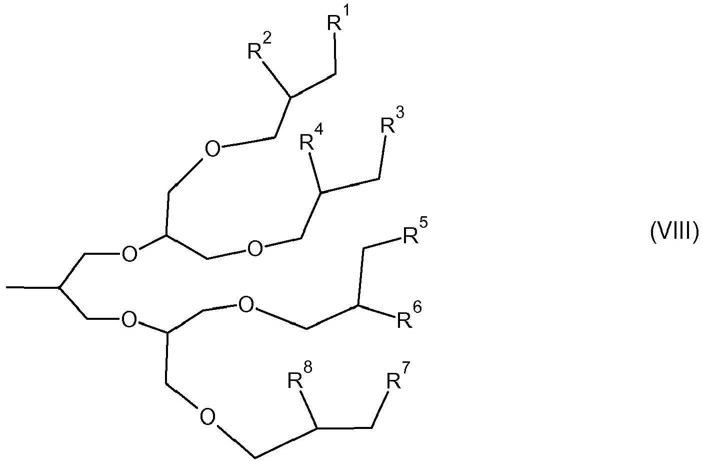 wherein R¹, R², R³, R⁴, R⁵, R⁶, R⁷, R⁸ is independently from each other -SH, -CH₃, -CH₂CH₃, -NH₂, -OH, -OCH₃, -OCH₂CH₃, -OCH₂OCH₂CH₃, -NH₃⁺, -OPO₃-(CH₂)₂N⁺(CH₃)₃, -(CH₃)₂N⁺(CH₂)₂OPO₂⁻(OCH₃), -(CH₃)₂N⁺(CH₂)₂OPO₂⁻(OH), or-OSO₃⁻, and X is O or S.

5. Diblock copolymer according to any of the preceding claims, wherein the superhydrophobic block comprises two carbon-containing chains that are bound to the same linker but are not covalently crosslinked to each other.

6. Diblock copolymer according to any of the preceding claims, wherein the superhydrophobic block comprises a perfluoroether residue.

7. Diblock copolymer according to any of the preceding claims, wherein the superhydrophobic block comprises a residue according to general formulae (IX) or (X), 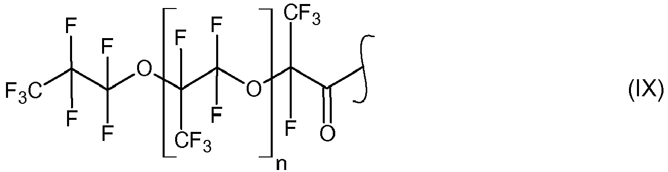 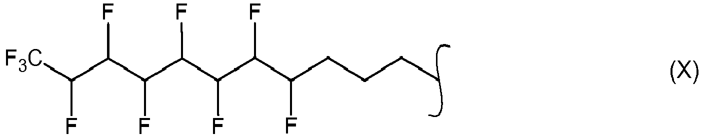 wherein n = 10 to 50.

8. Diblock copolymer according to any of the preceding claims, wherein the linker is chosen from the group consisting of amines, amides, oxygen, and an alkyl residue having 1 to 20 carbon atoms, wherein a hydrocarbon chain of the alkyl residue can be interrupted by one or more oxygen, nitrogen and/or sulfur atoms and/or can be substituted by a group comprising one or more oxygen, nitrogen and/or sulfur atoms.

9. Method for manufacturing a diblock copolymer, comprising reacting an oligoglycerol amine comprising protected hydroxyl groups with a perfluorinated polyether acid chloride in an organic solvent, and deprotecting the hydroxyl groups of the obtained diblock copolymer.

10. Method according to claim 9, wherein the oligoglycerol amine comprising protected hydroxyl groups is manufactured by mesylating an oligoglycerol comprising protected hydroxyl groups and at least one unprotected hydroxyl group to introduce an mesyl group into the oligoglycerol, substituting the mesyl group with an azide group and hydrogenating the azide group to obtain the oligoglycerol amine comprising protected hydroxyl groups.

11. Method according to claim 9 or 10, wherein the perfluorinated polyether acid chloride is manufactured by reacting a perfluorinated polyether with oxalyl chloride.

12. Droplet comprising a plurality of diblock copolymers according to any of claims 1 to 8.

13. Droplet according to claim 12, wherein it comprises a mixture of structurally different diblock copolymers.

14. Use of a droplet according to claim 12 or 13 for performing a PCR in an interior of the droplet.

15. Use of a droplet according to claim 12 or 13 for in vitro testing an effect of a substance on a cell incorporated in the droplet.
